# EUROPEAN PATENT SPECIFICATION

(11) **EP 4 346 636 B1**
(45) Date of publication and mention of the grant of the patent: **14.10.2026**
(21) Application number: 22727453.7
(22) Date of filing: 19.05.2022
(51) Int. Cl.: A61B 17/072

(54) **POWERED STAPLING DEVICE WITH MANUAL RETRACTION**
ELEKTRISCHES KLAMMERGERÄT MIT MANUELLEM RÜCKZUG
AGRAFEUSE MOTORISÉE AVEC RÉTRACTION MANUELLE

(30) Priority: 25.05.2021 US 202117329711
(43) Date of publication of application: 10.04.2024
(73) Proprietor: Covidien LP, Mansfield, MA 02048 (US)
(72) Inventor: CHEN, Xingrui, North Haven, Connecticut 06473 (US); CHOWANIEC, David, North Haven, Connecticut 06473 (US)
(74) Representative: Zimmermann & Partner Patentanwälte mbB
(86) International application number: PCT/IB2022/054686
(87) International publication number: WO 2022/248993

(56) References cited:
- US-A1- 2010 089 970
- US-A1- 2015 076 209
- US-A1- 2017 245 854
- US-A1- 2019 183 594

## Description

### FIELD

This disclosure is directed to surgical devices and, more particularly, to powered surgical stapling devices.

### BACKGROUND

Various types of surgical devices used to endoscopically treat tissue are known in the art, and are commonly used, for example, for closure of tissue or organs in transection, resection, and anastomoses procedures, for occlusion of organs in thoracic and abdominal procedures, and for electrosurgically fusing or sealing tissue.

One example of such a surgical device is a surgical stapling device. Typically, surgical stapling devices include a tool assembly having an anvil assembly and a cartridge assembly, and a drive assembly. Typically, the drive assembly includes a flexible drive beam and a clamp member that is supported on a distal end of the drive beam. The drive assembly is movable to advance the clamp member through the tool assembly to approximate the cartridge and anvil assemblies and to advance an actuation sled through the cartridge assembly to eject staples from the cartridge assembly.

Surgical stapling devices can be manually actuated devices in which a clinician squeezes a trigger to actuate the stapling device, or powered stapling devices in which a clinician activates a motor within the stapling device to actuate the stapling device. Although powered stapling devices require less force to operate, difficulties may arise when the device loses power or components of the device break. In such instances, the device can remain clamped about tissue preventing removal of the device from a patient.

A continuing need exists in the art for a powered stapling device that includes a drive assembly that can be manually retracted when power is lost or when the device is not operational. US 2010/089970 A1 relates to surgical cutting and fastening instruments and, more particularly, motor-driven surgical cutting and fastening instruments. It can include a motorized surgical cutting and fastening instrument having a drive shaft, a motor selectively engageable with the drive shaft, and a manual return mechanism configured to retract the drive shaft and operably disengage the motor from the drive shaft.

US 2015/076209 A1 relates to to powered surgical cutting and stapling instruments and staple cartridges that are designed to cut and staple tissue.

US 2017/245854 A1 relates to a surgical stapler for implanting mechanical surgical fasteners into the tissue of a patient, and, in particular, to a surgical stapler which is powered by a motor for firing surgical fasteners into tissue and a feedback controller for controlling the stapler in response to one or more sensed feedback signals.

### SUMMARY

The invention is defined in independent claim 1. Certain optional features of the invention are defined in the dependent claims.

A surgical device includes a powered handle assembly having a motor assembly, a rack, a spur gear, and a manual retract mechanism. The spur gear is movable from a position engaged with the motor assembly and the rack to a positioned disengaged from the motor assembly and engaged with the rack to facilitate manual retraction of the rack.

One aspect of the disclosure is directed to a powered handle assembly for a surgical device that includes a housing, a gear casing, a motor assembly, a rack, a rotating shaft, and a spur gear. The housing defines a cavity. The gear casing is supported within the cavity of the housing and defines a longitudinal channel, a first cavity, and a second cavity that communicate with each other. The motor assembly includes an output shaft and a drive gear that is secured to the output shaft. The motor assembly is secured to the gear casing, and the drive gear is positioned within the second cavity of the gear casing. The rack is received within the longitudinal channel of the gear casing and is movable between retracted and advanced positions. The rotating shaft extends through the first cavity of the gear casing. The spur gear is coupled to the rotating shaft and is received within the first cavity of the gear casing. The spur gear is movable within the first cavity from a first position in which the spur gear is engaged with the drive gear and the rack to a second position in which the spur gear is disengaged from the drive gear and engaged with the rack.

Other aspects of the disclosure are directed to a powered handle assembly for a surgical device that includes a housing, a motor assembly, a rack, a rotating shaft, and a spur gear. The housing defines a cavity. The motor assembly is supported within housing and includes an output shaft and a drive gear that is secured to the output shaft. The rack is supported within the housing and is movable longitudinally between retracted and advanced positions. The rotating shaft is supported within the housing. The spur gear is coupled to the rotating shaft and received within the housing such that the spur gear is movable from a first position in which the spur gear is engaged with the drive gear and the rack to a second position in which the spur gear is disengaged from the drive gear and engaged with the rack.

In aspects of the disclosure, the crank lever is coupled to the rotating shaft and is movable to move the spur gear from the first position to the second position.

In some aspects of the disclosure, a biasing member is engaged with the spur gear and urges the spur gear towards the first position.

In certain aspects of the disclosure, the rotating shaft includes a first portion and a second portion, wherein the first portion is rotatably fixed to the spur gear and the second portion receives the crank lever.

In aspects of the disclosure, the housing defines an opening and includes a removable cover that is positioned over the opening such that the crank lever is accessible through the opening.

In some aspects of the disclosure, the crank lever is movable along the second portion of the rotating shaft from a first position in which the rotating shaft can rotate independently of the crank lever to a second position in which the crank lever is rotatably fixed to the rotating shaft.

In certain aspects of the disclosure, the crank lever includes a hub that defines a through bore having a rectangular portion and the second portion of the rotating shaft includes a rectangular portion that is received within the rectangular portion of the through bore when the crank lever is in its second position.

In aspects of the disclosure, the handle assembly includes first and second C-clips, and the second portion of the rotating shaft defines spaced annular grooves that receive the first and second C-clips, respectively.

In some aspects of the disclosure, the spur gear is received about the second portion of the rotating shaft atop the first C-clip within the first cavity of the gear casing and the second C-clip is positioned externally of the first cavity of the gear casing to secure the rotating shaft to the gear casing.

Another aspect of the disclosure is directed to surgical stapling device that includes a powered handle assembly, an adapter assembly, and a tool assembly. The powered handle assembly includes a housing, a gear casing, a motor assembly, a rack, a rotating shaft, and a spur gear. The housing defines a cavity. The gear casing is supported within the cavity of the housing and defines a longitudinal channel, a first cavity, and a second cavity that communicate with each other. The motor assembly includes an output shaft and a drive gear secured to the output shaft. The motor assembly is secured to the gear casing, and the drive gear is positioned within the second cavity of the gear casing. The rack is received within the longitudinal channel of the gear casing and is movable between retracted and advanced positions. The rotating shaft extends through the first cavity of the gear casing. The spur gear is coupled to the rotating shaft and is received within the first cavity of the gear casing. The spur gear is movable within the first cavity from a first position in which the spur gear is engaged with the drive gear and the rack to a second position in which the spur gear is disengaged from the drive gear and engaged with the rack. The adapter assembly has a proximal portion coupled to the handle assembly and a distal portion. The adapter assembly includes a firing rod that is coupled to the rack and is movable between retracted and advanced positions in response to movement of the rack between its retracted and advanced positions. The tool assembly is supported on the distal portion of the adapter assembly.

In aspects of the disclosure, the tool assembly includes an anvil and a cartridge assembly that are movable between open and clamped positions.

In some aspects of the disclosure, the stapling device includes a drive assembly that is coupled to the firing rod and includes a working end having an I-beam configuration.

In certain aspects of the disclosure, the working end of the drive assembly is movable in relation to the anvil and the cartridge assembly in response to movement of the firing rod between its retracted and advanced positions.

Other aspects of the disclosure are directed to a powered handle assembly for a surgical device that includes a housing, a gear casing, a motor assembly, a drive screw, a drive nut, a connecting rod, and a spur gear. The housing defines a cavity. The gear casing is supported within the cavity of the housing and defines a channel. The motor assembly includes an output shaft and a drive gear secured to the output shaft. The motor assembly is secured to the gear casing, and the drive gear is positioned within the cavity of the gear casing. The drive screw is supported within the housing and is rotatable in response to activation of the motor assembly. The drive nut is supported on and movable along the drive screw between retracted and advanced positions. The connecting rod is coupled to the drive nut. The spur gear is movable within the channel of the gear casing from a first position in which the spur gear is engaged with the output shaft and the drive screw to a second position in which the spur gear is disengaged from the output shaft and engaged with the drive screw.

Other aspects of the disclosure are directed to a powered handle assembly for a surgical device that includes a housing, a motor assembly, a drive screw, a drive nut, a connecting rod, a spur gear, a locking clip, and a pawl assembly. The housing defines a cavity. The motor assembly includes an output shaft and a drive gear secured to the output shaft. The motor assembly is positioned within the housing and the drive gear is positioned within the cavity of the gear casing. The drive screw is supported within the housing and is rotatable in response to activation of the motor assembly. The drive nut is supported on and movable along the drive screw between retracted and advanced positions. The connecting rod is coupled to the drive nut. The spur gear is movable within the channel of the gear casing from a first position in which the spur gear is engaged with the output shaft and the drive screw to a second position in which the spur gear is disengaged from the output shaft and engaged with the drive screw. The locking clip is movable from a first position retaining the spur gear in its first position to a second position allowing movement of the spur gear from its first position to its second position. The pawl assembly includes an annular body portion and a ratcheting pawl coupled to the body portion.

In aspects of the disclosure, the handle assembly includes a biasing member that is positioned to urge the spur gear to the second position.

In some aspects of the disclosure, the handle assembly includes a locking clip that is movable from a first position retaining the spur gear in its first position to a second position allowing movement of the spur gear from its first position to its second position.

In certain aspects of the disclosure, the handle assembly includes a pawl assembly that includes a body portion and a ratcheting pawl coupled to the body portion.

In aspects of the disclosure, the gear casing defines a window and the body portion of the pawl assembly is positioned about the gear casing adjacent the window such that the pawl assembly is movable from a first position in which the ratcheting pawl is spaced from the spur gear to a second position in which the ratcheting pawl is engaged with the spur gear.

In some aspects of the disclosure, the connecting rod includes a proximal portion coupled to the drive nut and a distal portion coupled to a coupling member.

In certain aspects of the disclosure, the coupling member is adapted to engage a firing rod of the surgical device.

In aspects of the disclosure, the pawl assembly is coupled to the locking clip such that movement of the pawl assembly from its first position to its second position moves the locking clip from its first position to its second position.

In some aspects of the disclosure, the gear casing defines spaced openings, and the locking clip includes legs that are received within the openings.

Still other aspects of the disclosure are directed to a stapling device including a powered handle assembly, an adapter assembly, and a tool assembly. The powered handle assembly includes a housing, a gear casing, a motor assembly, a drive screw, a drive nut, a connecting rod, and a spur gear. the housing defines a cavity. The gear casing is supported within the cavity of the housing and defines a channel. The motor assembly includes an output shaft and a drive gear secured to the output shaft. The motor assembly is secured to the gear casing, and the drive gear is positioned within the cavity of the gear casing. The drive screw is supported within the housing and is rotatable in response to activation of the motor assembly. The drive nut is supported on and movable along the drive screw between retracted and advanced positions. The connecting rod is coupled to the drive nut. The spur gear is movable within the channel of the gear casing from a first position in which the spur gear is engaged with the output shaft and the drive screw to a second position in which the spur gear is disengaged from the output shaft and engaged with the drive screw. The adapter assembly has a proximal portion coupled to the handle assembly and a distal portion. The adapter assembly includes a firing rod that is coupled to the drive nut and is movable between retracted and advanced positions in response to movement of the drive nut between its retracted and advanced positions. The tool assembly is supported on the distal portion of the adapter assembly.

Other features of the disclosure will be appreciated from the following description.

### BRIEF DESCRIPTION OF THE DRAWINGS

Various embodiments of the disclosed staple cartridge are described herein below with reference to the drawings, wherein:
FIG. 1 is a side perspective view of a first version of a stapling device according to aspects of the disclosure with the stapling device in a non-articulated, unclamped position;
FIG. 2 is a side cutaway view of a handle assembly of the stapling device shown in FIG. 1 with a cover of the handle assembly removed;
FIG. 3 is a side perspective view of the handle assembly of the stapling device shown in FIG. 1 with a housing half-section removed;
FIG. 4 is an exploded side perspective view of internal components of the handle assembly shown in FIG. 3;
FIG. 5 is a side perspective view of a rotating shaft of the handle assembly shown in FIG. 3;
FIG. 6 is a perspective view of a crank lever of the handle assembly shown in FIG. 3;
FIG. 7 is a cross-sectional view taken along section line 7-7 of FIG. 2;
FIG. 8 is a cross-sectional view taken through a portion of the handle assembly shown in FIG. 3 with the stapling device in an unclamped position;
FIG. 9 is a cross-sectional view taken along section line 9-9 of FIG. 8;
FIG. 10 is a side perspective view of a reload assembly of the stapling device shown in FIG. 1 in the clamped and fired position;
FIG. 11 is a cross-sectional view taken along section line 11-11 of FIG. 10;
FIG. 12 is a cross-sectional view through a portion of the handle assembly shown in FIG. 3 with the stapling device in the clamped and fired position;
FIG. 13 is a side perspective view of a portion of the handle assembly shown in FIG. 3 as the stapling device is manually retracted from the clamped and fired position;
FIG. 14 is a cross-sectional view taken along section line 14-14 of FIG. 13;
FIG. 15 is a cross-sectional view taken along section line 15-15 of FIG. 14;
FIG. 16 is a side perspective view of the handle assembly of the stapling device shown in FIG. 1 with the cover of the handle assembly removed as the stapling device is manually retracted;
FIG. 17 is a cutaway, cross-sectional view taken through the handle assembly shown in FIG. 16 as the stapling device is manually retracted;
FIG. 18 is an alternate version of the handle assembly of the stapling device shown in FIG. 1 with a housing of the handle assembly shown in phantom;
FIG. 19 is an assembled view of the internal components of the handle assembly shown in FIG. 18;
FIG. 20 is an exploded side perspective view of internal components of the handle assembly shown in FIG. 19;
FIG. 21 is a side perspective view of a pawl assembly and locking clip of the handle assembly shown in FIG. 19;
FIG. 22 is a side perspective view from the proximal end of an interface between a motor assembly and a drive assembly of the handle assembly shown in FIG. 19 showing a manual retract mechanism with the locking clip in the locked position;
FIG. 23 is a side perspective view from the distal end of the interface between the motor assembly and the drive assembly of the handle assembly shown in FIG. 20 with a gear casing removed showing the manual retract mechanism with the locking clip in the locked position;
FIG. 24 is a cross-sectional view taken along section line 24-24 of FIG. 19;
FIG. 25 is a side cross-sectional view of the drive assembly of the handle assembly of the stapling device shown in FIG. 24 in the fired position;
FIG. 26 is a side perspective view from the proximal end of the interface between the motor assembly and the drive assembly of the handle assembly shown in FIG. 19 showing the manual retract mechanism with the locking clip in the unlocked position;
FIG. 26A is a side cross-sectional view taken along section line 26A-26A of FIG. 26 illustrating the manual retract mechanism in the unlocked position;
FIG. 27 is a cross-sectional view taken along section line 27-27 of FIG. 26 illustrating the manual retract mechanism as the manual retract mechanism is operated to move the locking clip from the locked position to the unlocked position; and
FIG. 28 is a cross-sectional view taken along section line 27-27 of FIG. 26 illustrating the manual retract mechanism as the manual retract mechanism is operated to retract the drive assembly.

### DETAILED DESCRIPTION

The disclosed surgical device will now be described in detail with reference to the drawings in which like reference numerals designate identical or corresponding elements in each of the several views. However, it is to be understood that the aspects of the disclosure are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure. In addition, directional terms such as front, rear, upper, lower, top, bottom, and similar terms are used to assist in understanding the description and are not intended to limit the disclosure.

In this description, the term "proximal" is used generally to refer to that portion of the device that is closer to a clinician, while the term "distal" is used generally to refer to that portion of the device that is farther from the clinician. In addition, the term "endoscopic" is used generally to refer to endoscopic, laparoscopic, arthroscopic, and/or any other procedure conducted through a small diameter incision or cannula. Further, the term "clinician" is used generally to refer to medical personnel including doctors, nurses, surgeons, and support personnel.

This disclosure is directed to a surgical device that includes a powered handle assembly having a motor assembly, a rack, a spur gear, and a manual retract mechanism. The spur gear is movable from a position engaged with the motor assembly and the rack to a positioned disengaged from the motor assembly and engaged with the rack to facilitate manual retraction of the rack.

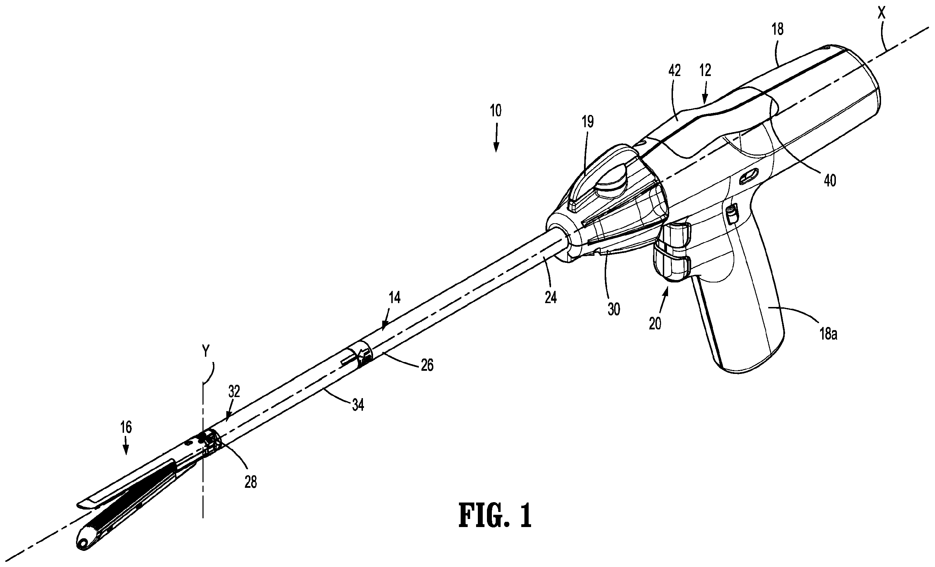

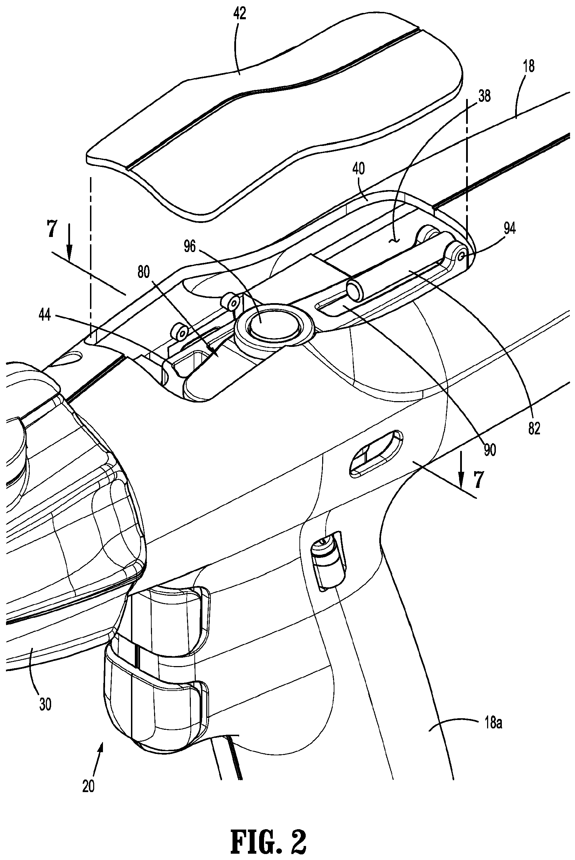

FIGS. 1 and 2 illustrate a surgical device shown generally as stapling device 10 which includes a handle assembly 12, an elongate body or adapter assembly 14, and a tool assembly 16. The handle assembly 12 includes a housing 18 that forms a stationary handle portion 18a, and articulation lever 19, and actuation buttons 20. The adapter assembly 14 defines a longitudinal axis "X" and includes a proximal portion 24 that is coupled to the handle assembly 12, and a distal portion 26 that supports the tool assembly 16. The tool assembly 16 is secured to the distal portion 26 of the adapter assembly 14 by a pivot member 28 that defines an axis "Y" that is transverse to the longitudinal axis "X". The articulation lever 19 is operatively coupled to the tool assembly 16 via an articulation linkage (not shown) such that manipulation of the articulation lever 19 causes articulation of the tool assembly 16 about the axis "Y" between an articulated position in which the tool assembly 16 is aligned with the longitudinal axis "Y" and non-articulated positions in which a longitudinal axis of the tool assembly and the longitudinal axis "X" define acute angles in response to manipulation of the articulation lever 19. The adapter assembly 14 is supported within a rotation knob 30 that is rotatably coupled to a distal portion of the handle assembly 12. The rotation knob 30 is manually rotatable about the longitudinal axis "X" to rotate the adapter assembly 14 and the tool assembly 16 about the longitudinal axis "X". The actuation buttons 20 control operation of the different functions of the stapling device 10 including clamping and firing of the stapling device 10.

In aspects of the disclosure, the tool assembly 16 forms part of a reload assembly 32 that includes a proximal body portion 34 and the tool assembly 16. The proximal body portion 34 of the reload assembly 32 forms an extension of the adapter assembly 14 and includes a proximal end that is adapted to be releasably coupled to a distal end of the adapter assembly 14 and a distal end that supports the tool assembly 16 for articulation. In aspects of the disclosure, the tool assembly 16 can be fixedly coupled to a distal portion of the adapter assembly 14.

In aspects of the disclosure, the housing 18 of the handle assembly 12 is formed from half-sections that are coupled together such as by welding of with screws to define a cavity 38 that receives internal components of the handle assembly 12 which are described in further detail below. The housing 18 defines an upper opening 40 that provides access to the internal components of the handle assembly 12. The upper opening 40 is enclosed by a cover 42 that is removably supported within the upper opening 40.

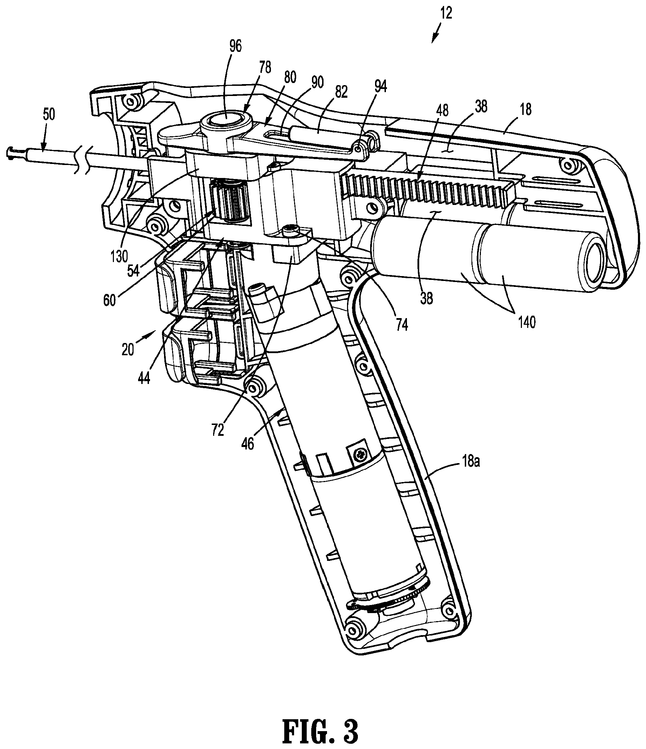

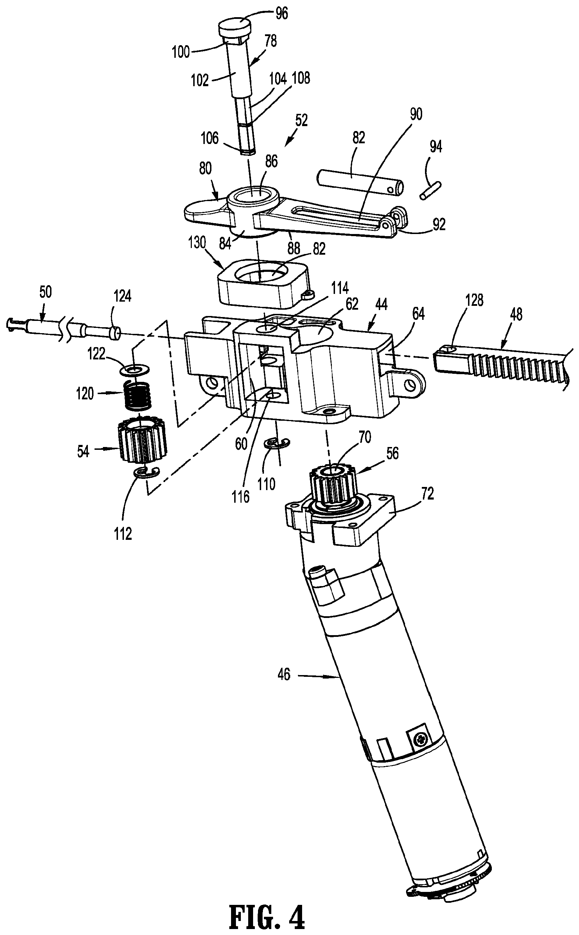

FIGS. 3 and 4 illustrate the internal components of the handle assembly 12 which include a gear casing 44, a motor assembly 46, a rack 48, a firing rod 50, a manual retract mechanism 52 and intermediate spur gear 54, and a drive gear 56. The gear casing 44 is secured within the cavity 38 of the housing 18 using screws or the like and defines a first cavity 60 and a second cavity 62 that intersect with each other and a longitudinally extending channel 64. The first cavity 60 of the gear casing 44 receives the drive gear 56 and the second cavity 62 of the gear casing 44 receives the intermediate spur gear 54. The drive gear 56 and the intermediate spur gear each include gear teeth that mesh such that rotation of the drive gear 56 within the first cavity 60 causes corresponding rotation of the intermediate spur gear 54 within the second cavity 62. The rack 48 is received within the channel 64 of the gear casing 44 and includes gear teeth that mesh with the gear teeth of the intermediate spur gear 54. When the drive gear 56 is rotated to rotate the intermediate spur gear 54, engagement between the intermediate spur gear 56 and the rack 48 causes the rack 48 to move longitudinally through the channel 64 in the gear casing 44.

The motor assembly 46 includes an output shaft 70 (FIG. 4) that is secured to the drive gear 56 and can be activated via the actuation buttons 20 (FIG. 1) to rotate the drive gear 56. In aspects of the disclosure, the motor assembly 46 is positioned within a portion of the cavity 38 of the housing 18 defined by the stationary handle portion 18a. The motor assembly 46 includes a mounting bracket 72 that is secured to the gear casing 44 with screws 74 such that the drive gear 56 is received within the second cavity 62 of the gear casing 44.

FIGS. 4-6 illustrate the manual retract mechanism 52 which includes a rotating shaft 78, a crank lever 80, and a grip member 82. The crank lever 80 includes a central hub portion 84 that defines a through bore 86 that receives the rotating shaft 78. The through bore 86 includes a cylindrical portion 86a and a rectangular portion 86b. The crank lever 80 also includes a lever portion 88 that defines a slot 90 and forms a clevis 92. The grip member 82 is supported within the clevis 92 by a pivot member 94 and is pivotable between a first position located within the slot 90 and a second position extending orthogonally from the lever portion 88.

The rotating shaft 78 includes a head portion 96 and a shaft portion 98 that extends downwardly from the head portion 96 as viewed in FIG. 4 through the through bore 86 in the crank lever 80. The shaft portion 98 includes a first rectangular portion 100, a cylindrical portion 102, and a second rectangular portion 104. The second rectangular portion 104 includes spaced annular grooves 106 and 108 that receive C-clips 110 and 112, respectively. The intermediate spur gear 54 is received about the second rectangular portion 104 of the rotating shaft 78 atop the C-clip 112 within the first cavity 60 of the gear casing 44. The C-clip 112 is positioned externally of the first cavity 60 and secures the rotating shaft 78 to the gear casing 44. Although the rotating shaft 78 is shown to have first and second rectangular portions, it is envisioned that other shaft configurations are envisioned.

The gear casing 44 defines first and second openings 114 and 116 that communicate with upper and lower ends of the first cavity 60 of the gear casing 44. The cylindrical portion 102 of the rotating shaft 78 is received within the first opening 114 of the gear casing 44 and the second rectangular portion 104 of the rotating shaft 78 extends through the second opening 116 of the gear casing 44. The C-clip 110 is secured to the lower end of the second rectangular portion 104 of the rotating shaft 78 adjacent a lower surface of the gear casing 44 and the C-clip 112 is secured to the second rectangular portion 104 of the rotating shaft 78 within the first cavity 60 of the gear casing 44. The C-clips 110 and 112 secure the intermediate spur gear 54 to the rotating shaft 78 and secure the rotating shaft 78 to the gear casing 44. The second rectangular portion 104 of the rotating shaft 78 is received within a rectangular bore in the intermediate spur gear 54 to rotatably fix the intermediate spur gear 54 to the rotating shaft 78.

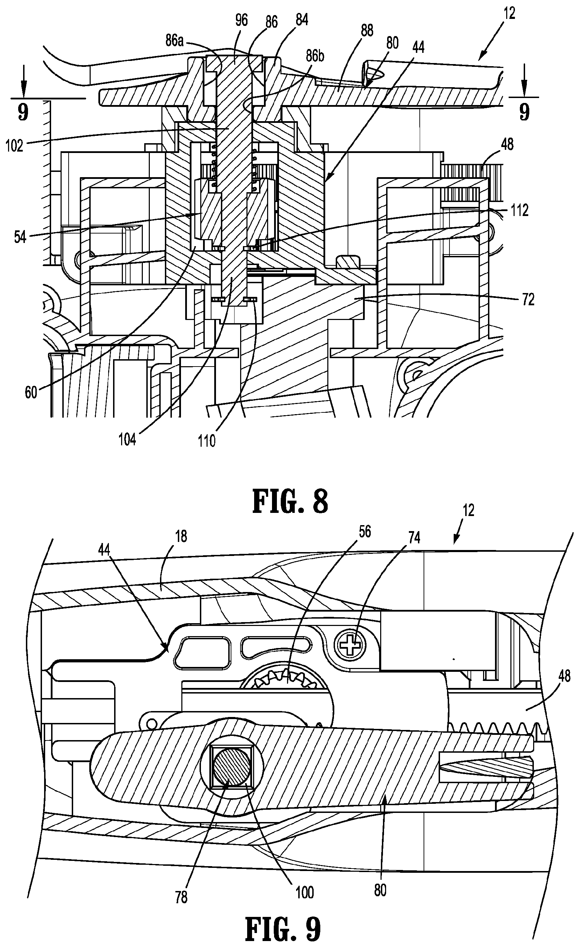

The intermediate spur gear 54 is received within the first cavity 60 of the gear casing 44 and is movable within the first cavity between a first or lower position and a second or upper position. In the lower position (FIG. 8), the teeth of the intermediate spur gear 54 simultaneously engage the teeth of the rack 48 and the teeth of the drive gear 56. In the upper position, the teeth of the intermediate spur gear 54 engage only the teeth of the rack 48.

The manual retract mechanism 52 includes a biasing member 120 that is positioned between an upper surface of the intermediate spur gear 54 and the inner surface of the gear casing 44. The biasing member120 urges the intermediate spur gear 54 towards its lower position in which the intermediate spur gear 54 is engaged with both the drive gear 56 and the rack 48. In some aspects of the disclosure, a washer 122 is positioned between an upper surface of the biasing member 120 and the inner surface of the gear casing 44. In some aspects of the disclosure, the biasing member 120 includes a coil spring. It is envisioned that other types of biasing members could be incorporated into the stapling device 10 (FIG. 1).

The rack 44 includes a distal portion that is coupled to the firing rod 50, In aspects of the disclosure, the proximal portion of the firing rod 50 is formed with a head 124 that has a diameter that is larger than a body of the firing rod 50 and the distal portion of the rack 48 defines a slot 128. The head 124 of the firing rod 50 is received within the slot 128 of the rack 48 to couple the firing rod 50 to the rack 48 such that longitudinal movement of the rack 48 causes longitudinal movement of the firing rod 50. The coupling of the head 124 and the slot 128 allows the firing rod 50 to rotate in relation to the rack 48.

In some aspects of the disclosure, the manual retract mechanism 52 includes a base member 130 that defines a circular bore 132. The base member 130 is secured to an upper surface of the gear casing 44 and the hub portion 84 of the crank lever 80 is aligned within the circular bore 132. The crank lever 80 is movable from a lowered position to a raised position. In the lowered position, the hub portion 84 of the crank lever 80 is received within the circular bore 132 and in the raised position, the hub portion 84 of the crank lever 80 is positioned above the circular bore 132 of the base member 130.

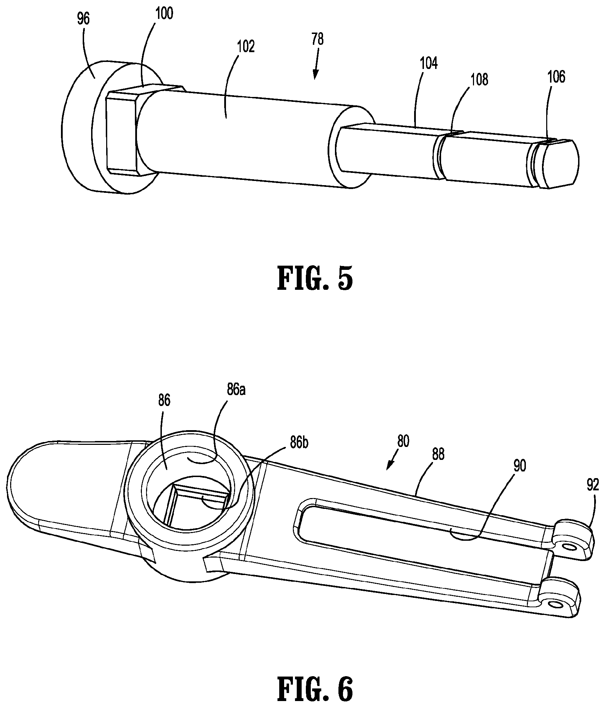

The head 96 of the rotating shaft 78 of the manual retract mechanism 52 is received within the bore 86 of the crank lever 80. When the crank lever 80 is in its lowered position, the cylindrical portion 102 of the rotating shaft 78 is received within the rectangular portion 86b (FIG. 6) of the through bore 86 of the crank lever 80. In this position, the rotating shaft 78 can rotate within the through bore 86 of the crank lever 80 without causing rotation of the crank lever 80. When the crank lever 80 is moved to its raised position, the first rectangular portion 100 of the rotating shaft 78 is received within the rectangular portion 86b of the through bore 86 of the crank lever 80, In this position, rotation of the crank lever 80 causes corresponding rotation of the rotating shaft 78, and thus, rotation of the intermediate spur gear 54. The head 96 of the rotating shaft 78 has a diameter that is larger than the width of the rectangular portion 86b of the through bore 86 of the crank lever 80. Thus, movement of the crank lever 80 to its raised position causes the rotating shaft 78 to its upper position in which the teeth of the intermediate spur gear 54 are only engaged with the teeth of the rack 48.

The handle assembly 12 (FIG. 1) also includes one or more batteries 140 which are received within the cavity 38 of the housing 18 of the handle assembly 12. The batteries 140 provide power to the motor assembly 46 via the actuation switches and control circuitry (not shown), e.g., a printed circuit board and one or more controllers, within the handle assembly 12 to control firing of the stapling device 10.

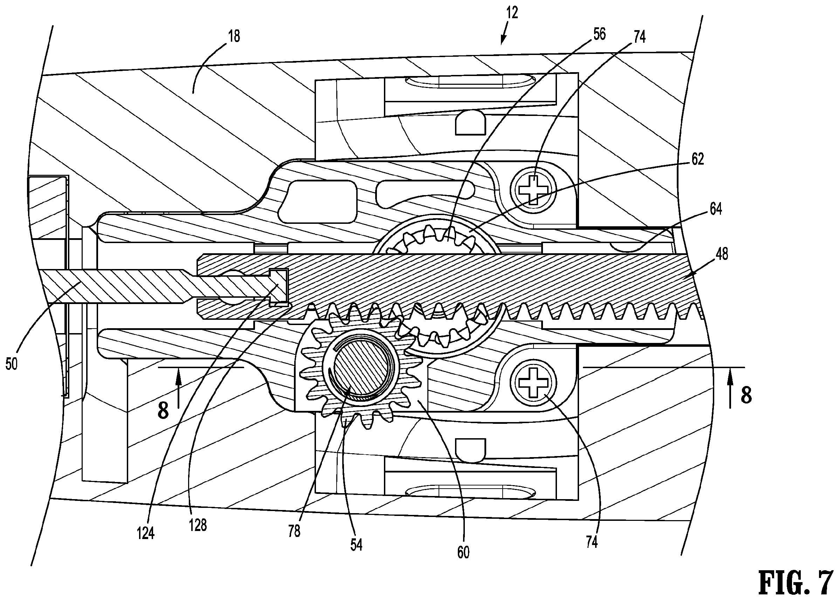

FIGS. 7-9 illustrate the handle assembly 12 of the stapling device 10 (FIG. 1) with the stapling device in an unclamped position prior to firing of the stapling device. In this position, the rack 48 is in a retracted position within the channel 64 of the gear casing 44 and the intermediate spur gear 54 is in its lowered position and is engaged with the rack 48 and the drive gear 56.

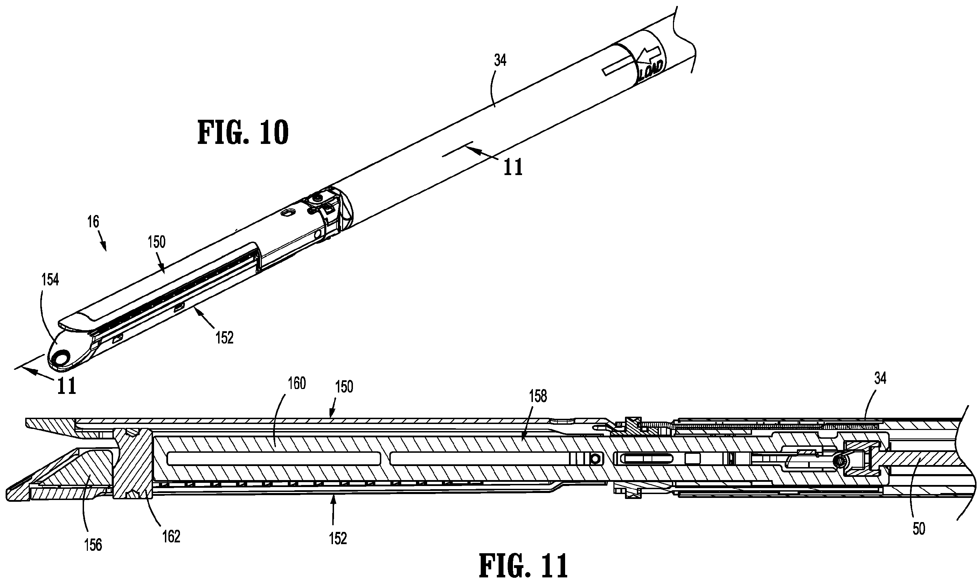

FIGS. 10 and 11 illustrate the reload assembly 32 when the stapling device 10 is in a fired position. As described above, the reload assembly 32 includes the tool assembly 16 and the proximal body portion 34. In aspects of the disclosure, the tool assembly 16 includes an anvil assembly 150 and a cartridge assembly 152. The cartridge assembly 152 includes a staple cartridge 154 that supports a plurality of staples and pushers (not shown), and an actuation sled 156. The proximal body portion 32 includes a drive assembly 158 that includes a flexible beam 160 and a working end 162. The working end 162 of the drive assembly 158 has an I-beam configuration and is secured to a distal end portion of the flexible beam 160. The flexible beam 160 has a proximal end portion that is releasably coupled to a distal portion of the firing rod 50. When the firing rod 50 is moved from a retracted position to an advanced position, the drive assembly 158 moves from a retracted position to an advanced position to move the working end 162 of the drive assembly 158 through the tool assembly 16 to advance the actuation sled 156 through the tool assembly 16. As the actuation sled 156 moves through the tool assembly 16, the actuation sled 156 engages the pushers (not shown) to eject staples (not shown) from the staple cartridge 154 into the anvil assembly 150. In the fired position, the working end 162 of the drive assembly 158 and the actuation sled 156 are in their advanced positions within the tool assembly and the tool assembly is in the clamped position clamped about tissue (not shown). For a more detailed description of the operation of the drive assembly 158 and its interaction with the tool assembly, see U.S. Patent No. 8,132,706.

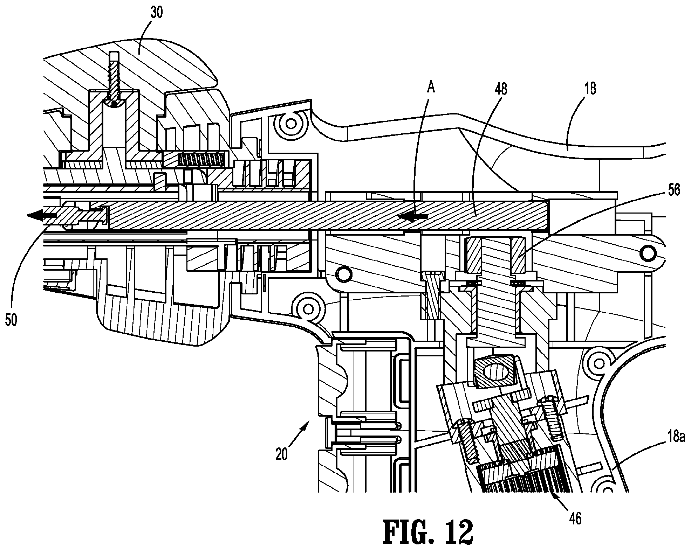

FIG. 12 illustrates the handle assembly 12 of the stapling device 10 (FIG. 1) as the stapling device 10 is fired. When the stapling device 10 is fired, the intermediate spur gear 54 (FIG. 8) is engaged with the rack 48 and with the drive gear 56. When the motor assembly 46 is activated, the drive gear 56 rotates the intermediate spur gear 54 to advance the rack 48 in the direction indicated by arrows "A". The rack 48 is coupled to the firing rod 50 such that advancement of the firing rod 50 advancement of the rack 48 causes advancement of the firing rod 50 in the direction of arrows "A" to advance the drive assembly 158 (FIG. 11) within the tool assembly 16.

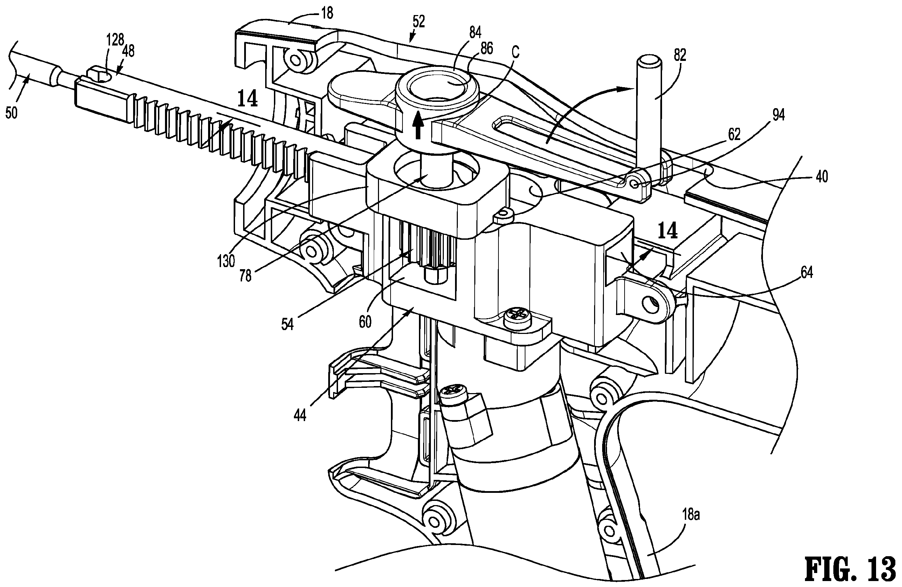

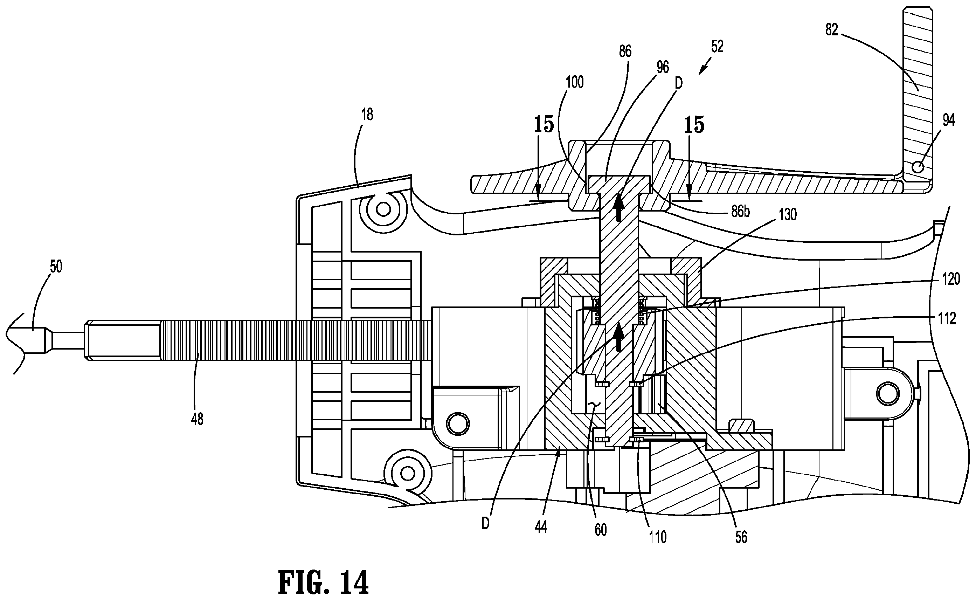

FIGS. 13-15 illustrate the manual retract mechanism 52 as it is readied for use. When the stapling device 10 loses power or gets damaged such that the motor assembly 46 cannot retract the drive assembly 158 to release tissue clamped between the anvil and cartridge assemblies 150 and 152 (FIG. 11), the manual retract mechanism 52 can be operated to retract the drive assembly 158 (FIG. 11). In order to access the manual retract mechanism 52, the cover 42 (FIG. 2) must be removed to uncover the upper opening 40 in the housing 18 of the handle assembly 12. Once the cover is removed, the crank lever 80 is pulled upwardly in the direction of arrow "C" in FIG. 13 to move the crank lever 80 from its lowered position to its raised position. As the crank lever 80 is moved towards its raised position, the first rectangular portion 100 of the rotating shaft 78 is received in the rectangular portion 86b of the through bore 86 in the crank lever 80. Once the first rectangular portion 100 of the rotating shaft 78 is received in the rectangular portion 86b of the through bore 86 in the crank lever 80, continued movement of the crank lever 80 in the direction of arrow "C" will lift the rotating shaft 78 and the intermediate spur gear 54 in the direction of arrows "D" in FIG. 14 to their upper positions compressing the biasing member 120. In the upper position, the intermediate spur gear 54 is disengaged from the drive gear 56 and is engaged only with the rack 48. Once the crank lever 80 is in its raised position, the grip member 82 can be pivoted about the pivot member 94 to an operational position.

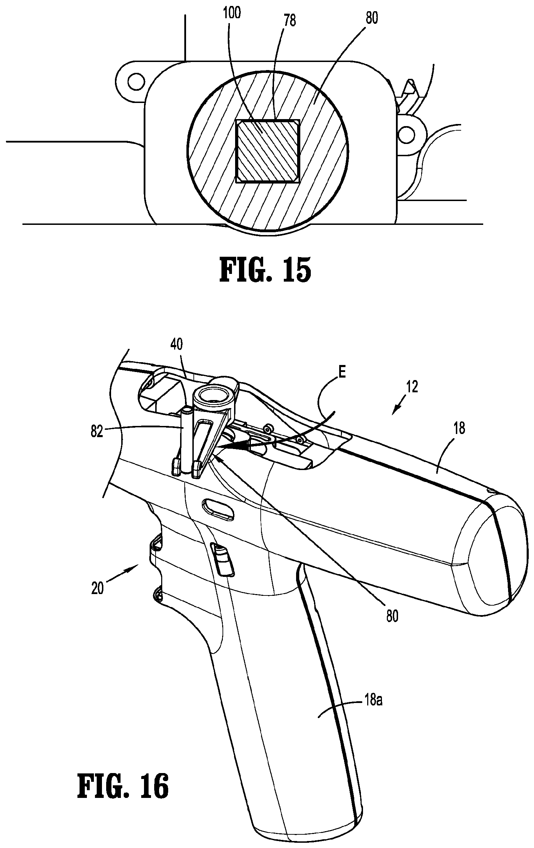

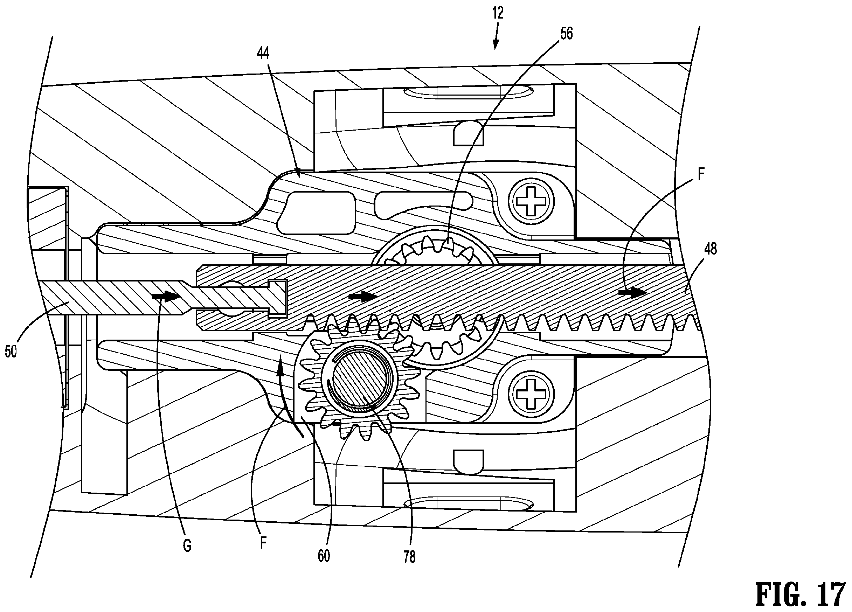

FIGS. 16 and 17 illustrate the manual retract mechanism 52 as it is operated to retract the firing rod 50. Once the crank lever 80 is moved to its raised position, the crank lever 80 can be rotated in the direction indicated by arrow "E" in FIG. in FIG. 16 to rotate the rotating shaft 78 and the intermediate spur gear 54 in the direction indicted by arrow "F" in FIG. 17 to retract the rack 48. More specifically, when the crank lever 80 is rotated, receipt of the first rectangular portion 100 of the rotating shaft 78 in the rectangular portion 86b of the through bore 86 of the crank lever 80 rotatably fixes the crank lever 80 to the rotating shaft 78. Thus, when the crank lever 80 rotates, the rotating shaft also rotates. The intermediate spur gear 54 is rotatably fixed to the rotating shaft 78 via receipt of the second rectangular portion 104 of the rotating shaft 78 in the rectangular bore 54a of the intermediate spur gear 54 such that rotation of the rotating shaft 78 causes rotation of the intermediate spur gear 54. In its upper position, the intermediate spur gear 54 is only engaged with the rack 48, and as such, rotation of the intermediate spur gear 54 causes retract of the rack 48.

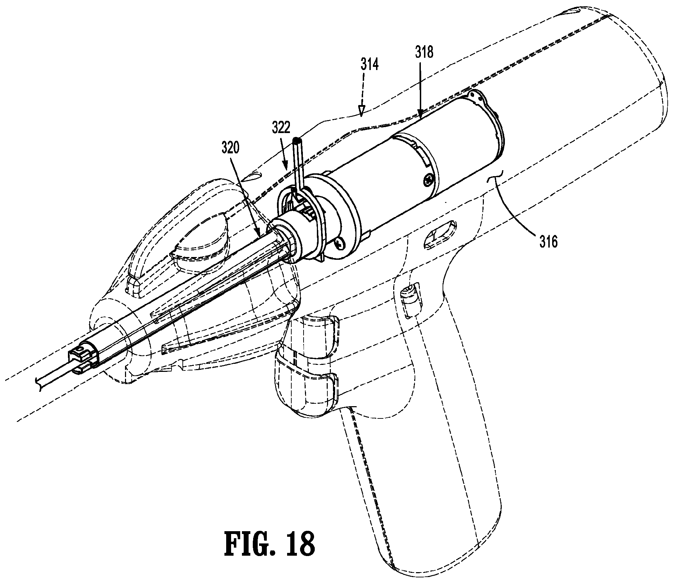

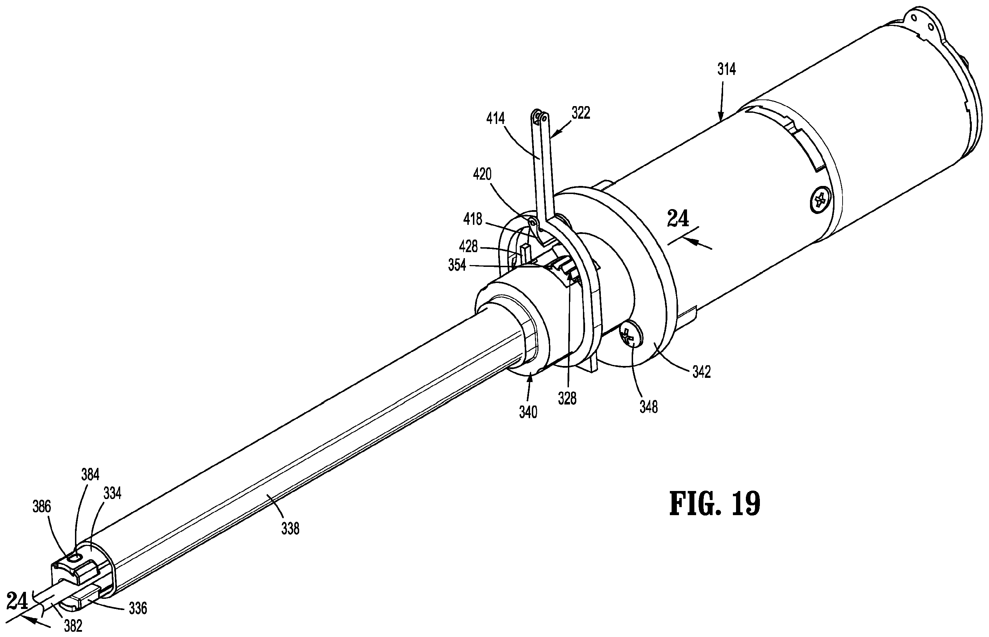

FIGS. 19-28 illustrate an alternate version of the handle assembly 12 (FIG. 18) of the stapling device 10 shown generally as handle assembly 312. The handle assembly 312 includes a housing 314 that is substantially similar to housing 18 (FIG. 1) of stapling device 10 and will not be described in further detail herein. The housing 314 defines a cavity 316 that receives the internal components of the handle assembly 312.

FIGS. 18-20 illustrate the internal components of the handle assembly 312 which includes a motor assembly 318, a drive assembly 320, and a manual retract mechanism 322. The motor assembly 318 is supported within the cavity 316 (FIG. 18) of the housing 314 and includes an output shaft 324 that has a flat surface 324a. In some aspects of the disclosure, the output shaft 324 has a D-shaped configuration although other configurations are envisioned.

The drive assembly 320 is coupled to the output shaft 324 of the motor assembly 318 and includes a one-way spur gear 328, a drive screw 330, a drive nut 332, connecting rods 334, a coupling member 336, a guide tube 338, and a gear casing 340. The gear casing 340 includes a mounting flange 342 and a cylindrical body 344. The mounting flange 342 of the gear casing 340 defines bores 346 that receive screws 348. The screws 348 are received in threaded bores 350 formed in a distal face of the motor assembly 318 to secure the gear casing 340 to the motor assembly 318. The cylindrical body 344 of the gear casing 340 defines a cavity 352 and a window 354 that communicates with the cavity 352. The cylindrical body 344 of the gear casing 340 defines two openings 356 (only one is shown) and two cutouts 358. One of the openings 356 and one of the cutouts 358 is positioned on each side of the window 354 in vertical alignment. The cavity 352 of the cylindrical body 344 of the gear casing 340 receives the one-way spur gear 328. The distal portion of the cylindrical body 344 of the gear casing 340 supports a bearing 359.

The one-way spur gear 328 defines a central through bore 360 that receives a bearing 362. In aspects of the disclosure, the central through bore 360 and the bearing 362 have corresponding non-circular configurations, e.g., D-shaped configurations, such that the bearing 362 is slidably received within the central through bore 360 of the one-way spur gear 328. The corresponding configurations of the one-way spur gear 328 and the bearing 362 rotatably fix the components to each other. The bearing 362 also defines a central through bore 364 that has a non-circular configuration.

The drive screw 330 includes a threaded outer surface 366, a proximal extension 368, and a distal extension 370. The proximal extension 368 of the drive screw 330 extends through the bearing 359 within the gear casing 340 and is received and secured within the central through bore 364 of the bearing 364. The distal extension 370 of the drive screw 330 is received within a bearing 372 that is supported within the housing 314 (FIG. 18) to rotatably support the drive screw 330 within the housing 314.

When the motor assembly 318 is activated to rotate the output shaft 324, rotation of the output shaft 324, when engaged with the one-way spur gear 328, causes corresponding rotation of the one-way spur gear 328. As described above, the one-way spur gear 328 is rotatably fixed to the bearing 362 which is secured to and rotatably fixed to the drive screw 330. As such, rotation of the one-way spur gear 328 causes corresponding rotation of the drive screw 330.

The drive nut 332 includes a threaded bore 374 that receives and is threadably engaged with the threaded outer surface 366 of the drive screw 330. The drive nut 332 is coupled to a proximal portion of the connecting rods 334. In aspects of the disclosure, the drive nut 332 includes protrusions 378 that are received within openings 380 formed in the proximal portions of the connecting rods 334 to connect the drive nut 332 to the connecting rods 334. The connecting rods 334 extend distally from the drive nut 332 and include distal portions that are connected to the coupling member 336. In aspects of the disclosure, the coupling member 336 includes protrusions 384 that are received within openings 386 formed in the distal portions of the connecting rods 334 to connect the coupling member 336 to the connecting rods 334. The drive nut 332 and the connecting rods 334 are received within the guide tube 338.

When the drive screw 330 is rotated, engagement between the outer threaded surface 366 of the drive screw 330 and the inner threaded bore 374 of the drive nut 332 causes the drive nut 332 to translate longitudinally along the drive screw 330 within the guide tube 338. The drive nut 332 is connected to the connecting rods 334 such that longitudinal translation of the drive nut 332 along the drive screw 330 causes the connecting rods 334 to move longitudinally within the guide tube to advance to coupling member 336.

The coupling member 336 is coupled to a firing rod 382 such that longitudinal movement of the coupling member 336 causes longitudinal movement of the firing rod 382. In aspects of the disclosure, the firing rod 382 includes a head portion 384 and an elongate body 386. The head portion 384 has a diameter that is greater than a diameter of the elongate body 386. The coupling member 336 defines a slot 390 that has a width that is greater than the diameter of the elongate body 386 but less than the diameter of the head portion 384. The elongate body 386 of the firing rod 382 is received through the slot 390 in the coupling member 336 to axially fix the firing rod 382 to the coupling member 336 while allowing relative rotation of the firing rod 382 and the coupling member 336.

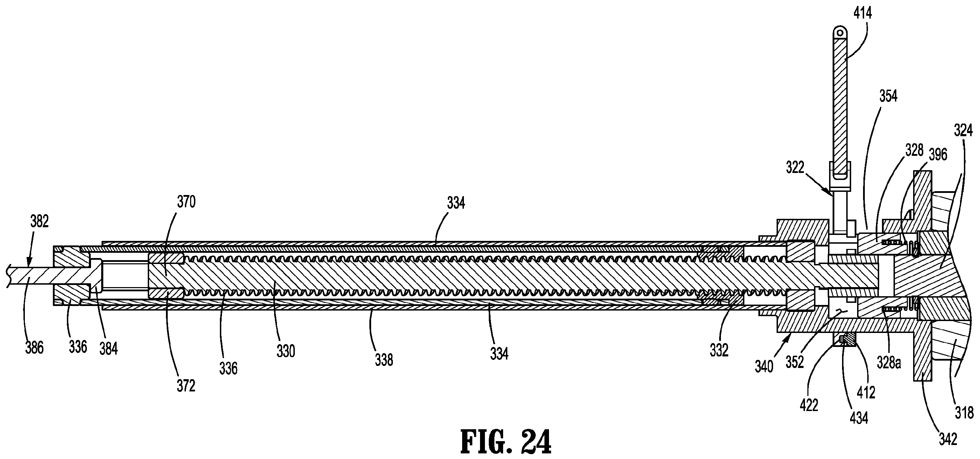

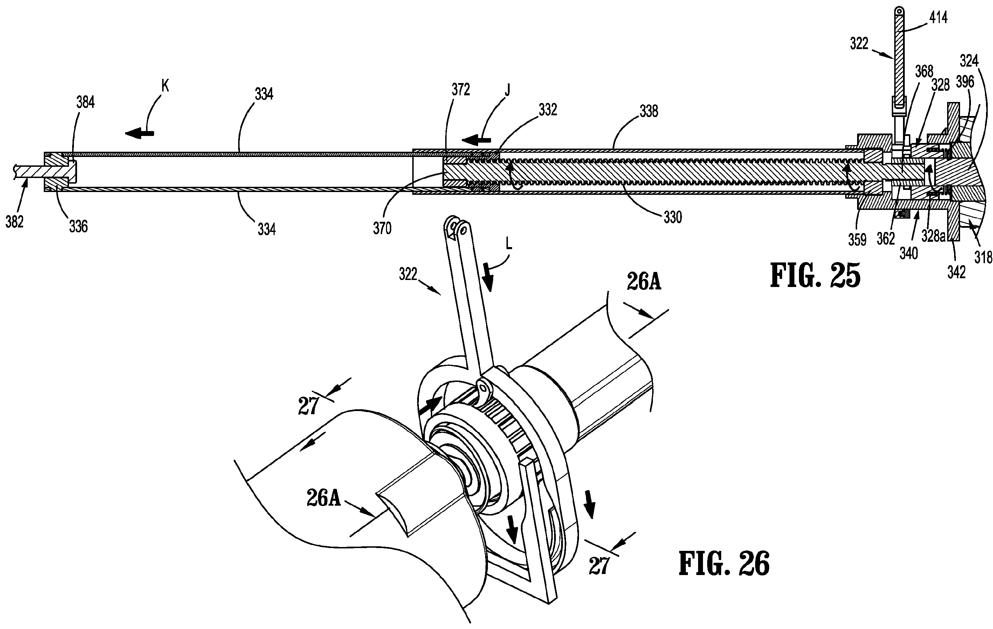

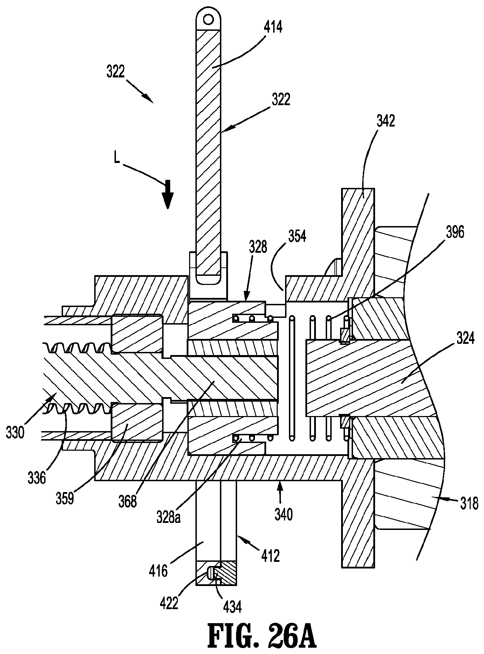

The one-way spur gear 328 is movably positioned within the cavity 352 of the cylindrical body 344 of the gear casing 340 between a retracted position (FIG. 24) and an advanced position (FIG. 26A). In the advanced position, the one-way spur gear is engaged with the proximal extension 368 of the drive screw 330 and the output shaft 324 of the motor assembly 318 such that rotation of the output shaft 324 of the motor assembly 324 causes rotation of the drive screw 330. In the advanced position, the one-way spur gear 328 is disengaged from the output shaft 324 of the motor assembly 318 but still engaged with the drive screw 330. A biasing member 396, e.g., a coil spring, is positioned between the distal surface of the motor assembly 318 and a proximal surface of the one-way spur gear 328 to urge the one-way spur gear 328 towards the advanced position. In aspects of the disclosure, the proximal surface of the one-way spur gear 328 defines a recess 328a (FIG. 26A) that receives the biasing member 396.

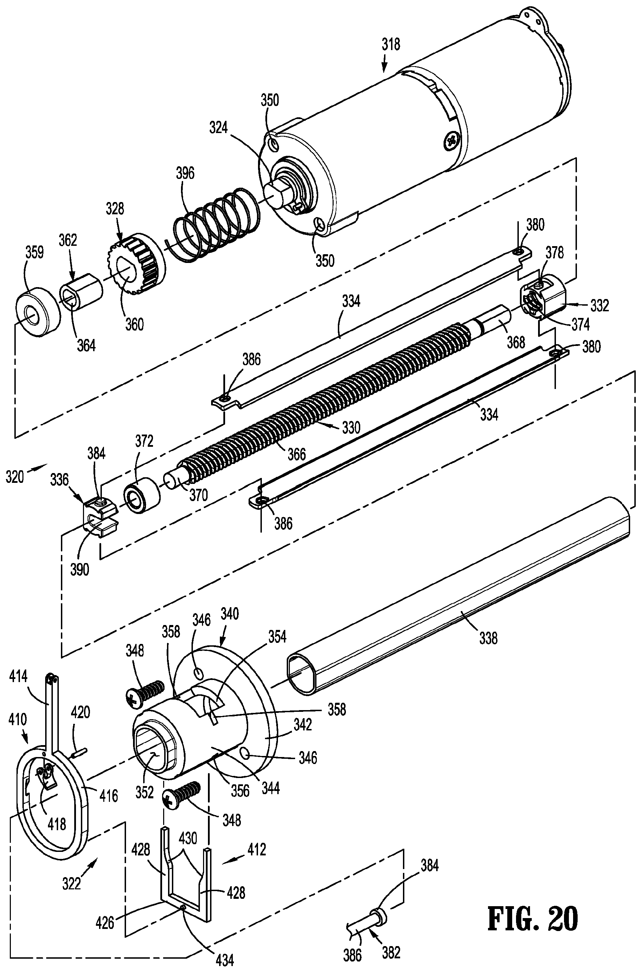

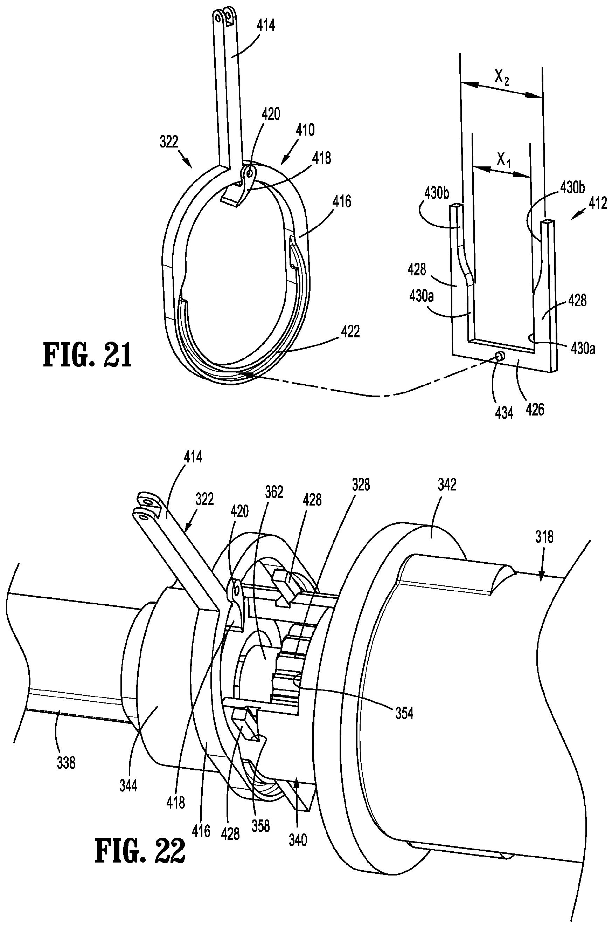

FIGS. 20 and 21 illustrate the manual retract mechanism 322 includes a pawl assembly 410 and a locking clip 412. The pawl assembly 410 includes a handle 414 and a body portion 416. In aspects of the disclosure, the body portion has an oval or annular configuration and supports a ratcheting pawl 418 that is pivotably secured to an upper portion of the body portion 416 by a pivot member 420. The ratcheting pawl 418 extends downwardly into a circular opening defined by the body portion 416. The body portion 416 is received about the gear casing 340 with the ratcheting pawl 418 positioned over the window 354 in the gear casing 340 above the one-way spur gear 328. A lower portion of the body portion 416 defines a circular slot 422.

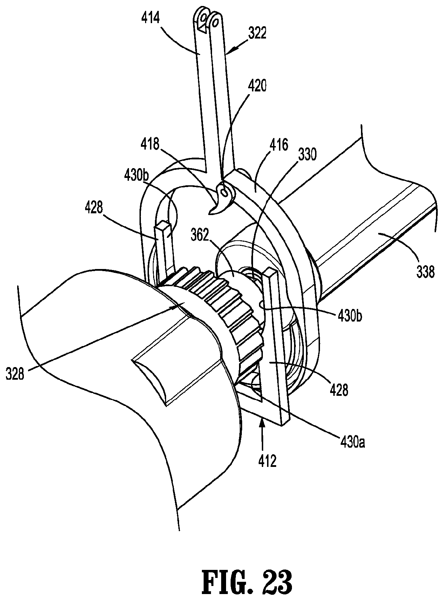

The locking clip 412 has a rectangular shape and includes a base portion 426 and spaced legs 428 that extend upwardly from the base portion 426. Each of the legs 428 of the locking clip 412 includes a stepped inner surface 430 that includes a first surface 430a and a second surface 430b. The first surfaces 430a of the legs 428 define a first width X₁ and the second surfaces 430b define a second width X₂ that is greater than the first width X₂. Each of the legs 428 is received through one of the openings 356 and cutouts 358 of the gear casing 340 such that the stepped inner surfaces 430 of the legs 428 of the locking clip 412 are positioned within the cavity 352 of the gear casing 340. The locking clip 322 is movable from a first position (FIG. 23) in which the first surfaces 430a of the legs 428 of the locking clip 322 are aligned with the one-way spur gear 328 and a second position in which the second surfaces 430b of the legs 428 of the locking clip 322 are spaced from the one-way spur gear 328. The width X₁ between the first surfaces 430a of the legs 428 of the locking clip 322 is such to prevent movement of the one-way spur gear 322 to its advanced position, whereas the width X₂ between the second surfaces 430b of the legs 428 of the locking clip 322 allows movement of the one-way spur gear 322 to the advanced position.

The base portion 426 of the locking clip 322 includes a protrusion 434 that is received within the circular slot 422 in the body portion 416 of the pawl assembly 410. Receipt of the protrusion 434 couples the pawl assembly 410 to the locking clip 322 to retain the pawl assembly 410 in a stable position about the gear casing 340. In aspects of the disclosure, the protrusion 434 has an enlarged head and the circular slot includes overhanging ledges that retain the enlarged head of the protrusion 434 within the circular slot 422. The protrusion 434 is configured to slide within the circular slot 422 as described in further detail below.

FIGS. 22-24 illustrate the handle assembly 12 (FIG. 18) in a pre-fired position with the pawl assembly 322 positioned about the gear casing 340 and the ratcheting pawl 418 positioned above the window 354 in the gear casing 340. When the handle assembly 12 is assembled, the one-way spur gear 328 is pressed proximally towards the motor assembly 318 to compress the biasing member 396 and position the one-way spur gear 328 in its retracted position. After the one-way spur gear 328 is in its retracted position, the legs 428 of the locking clip 412 are inserted from a side of the gear casing 340 opposite to the ratcheting pawl 418 into the openings 356 and cutouts 358 formed in the gear casing 340 to its first position. In the first position of the locking clip 412, the first surfaces 430a of the legs 428 of the locking clip 412 engage a distal face of the one-way spur gear 328 to retain the one-way spur gear 328 in its retracted position against the urging of the biasing member 396. In its retracted position, the one-way spur gear 328 is engaged with both the output shaft 324 of the motor assembly 318 and the one-way spur gear 328. When the locking clip 412 is in its first position, the protrusion 434 on the locking clip 412 is pressed into the circular slot 422 on the body portion 416 of the pawl assembly 322 to couple the pawl assembly 322 to the locking clip 412 (FIG. 24).

In the pre-fired position of the handle assembly 12 (FIG. 18), the drive nut 322 is positioned near the proximal end of the drive screw 330 and the coupling member 336 is positioned adjacent the distal end of the guide tube 338 such that the connecting rods 334 are in retracted positions and the firing rod 382 is in its retracted position.

FIG. 25 illustrates the handle assembly 12 (FIG. 1) in a fired position. When the stapling device 10 (FIG. 1) is fired by pressing the actuation buttons 20 (FIG. 1), the motor assembly 318 is activated to rotate the output shaft 324. Rotation of the output shaft 324 causes corresponding rotation of the one-way spur gear 328 and the drive screw 330 to advance the drive nut 322 along the drive screw 330 in the direction of arrow "J". As the drive screw 330 advances the drive nut 322, the drive nut 322 advances the connecting rods 334 to advance the firing rod 382 in the direction of arrow "K" and actuate the tool assembly 16 (FIG. 1) as described above regarding stapling device 10 (FIG. 1).

When the tool assembly 16 is in the clamped and fired position (FIG. 11) and the powered stapling device 10 (FIG. 1) becomes inoperable and cannot be unclamped using the motor assembly 318, the manual retract mechanism 322 allows the tool assembly to be manually unclamped. FIGS. 26-28 illustrate operation of the manual retract mechanism 322. In order to operate the manual retract mechanism 322, the pawl assembly 322 is pressed downwardly in the direction of arrows "L" in FIGS. 26-27. When the pawl assembly 322 is pressed downwardly, the locking clip 412, which is coupled to the pawl assembly 322 by the protrusion 434, is moved from its first position to its second position. In its second position, the locking clip 412 disengages from the one-way spur gear 328 such that the biasing member 396 moves the one-way spur gear 328 from its retracted position to its advanced position. In its advanced position, the one-way spur gear 328 is disengaged from the output shaft 324 of the motor assembly 318. When the pawl assembly 322 is pressed downwardly, the ratcheting pawl 418 moves through the window 354 of the gear housing 340 into engagement with the one-way spur gear 328.

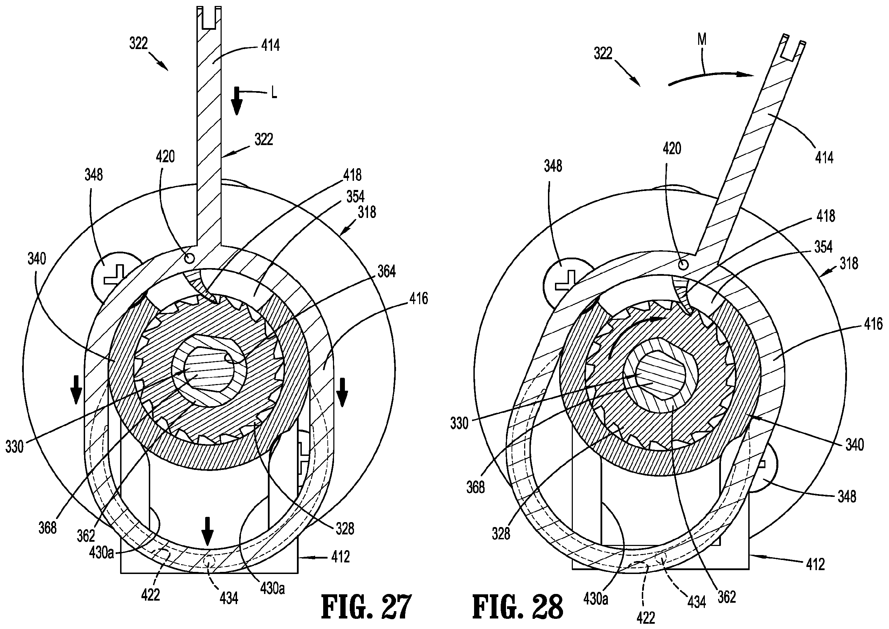

After the one-way spur gear 328 is in its advanced position, the handle 414 of the pawl assembly 322 can be rotated in the direction of arrow "M" in FIG. 28 to rotate the one-way spur gear 328 and the drive screw 330 to retract the firing rod 382. As the pawl assembly 322 is rotated, the protrusion 434 moves within the circular slot 422 of the pawl assembly 322.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the disclosure. As well, one skilled in the art will appreciate further features and advantages of the disclosure based on the above-described embodiments. Accordingly, the disclosure is not to be limited by what has been particularly shown and described, except as indicated by the appended claims.

## Claims

1. A powered handle assembly (12) for a surgical device (10) comprising:
a housing (12) defining a cavity (38);
a gear casing (44) supported within the cavity of the housing, the gear casing defining a longitudinal channel (64), a first cavity (60), and a second cavity (62) that communicate with each other;
a motor assembly (46) including an output shaft (70) and a drive gear (56) secured to the output shaft, the motor assembly secured to the gear casing, and the drive gear being positioned within the second cavity of the gear casing;
a rack (48) received within the longitudinal channel of the gear casing and movable between retracted and advanced positions;
a rotating shaft (78) extending through the first cavity of the gear casing; and
a spur gear (54) coupled to the rotating shaft and received within the first cavity of the gear casing, the spur gear movable within the first cavity from a first position in which the spur gear is engaged with the drive gear and the rack to a second position in which the spur gear is disengaged from the drive gear and engaged with the rack.

2. The powered handle of claim 1, further including a crank lever (80) coupled to the rotating shaft, the crank lever movable to move the spur gear from first position to the second position, optionally further including a biasing member (120) engaged with the spur gear, the biasing member urging the spur gear towards the first position.

3. The powered handle of claim 2, wherein the rotating shaft includes a first portion (104) and a second portion (108), the first portion rotatably fixed to the spur gear, and the second portion receiving the crank lever.

4. The powered handle of claim 3, wherein the housing defines an opening (40) and includes a removable cover (42) positioned over the opening, the crank lever being accessible through the opening.

5. The powered handle of claim 4, wherein the crank lever is movable along the second portion of the rotating shaft from a first position in which the rotating shaft can rotate independently of the crank lever to a second position in which the crank lever is rotatably fixed to the rotating shaft.

6. The powered handle of claim 5, wherein the crank lever includes a hub (84) that defines a through bore having a rectangular portion (86b) and the second portion of the rotating shaft includes a rectangular portion (104) that is received within the rectangular portion of the through bore when the crank lever is in its second position.

7. The powered handle of claim 3, further including first and second C-clips (110, 112), the second portion of the rotating shaft defining spaced annular grooves that receive the first and second C-clips, respectively, wherein the spur gear is received about the second portion of the rotating shaft atop the first C-clip within the first cavity of the gear casing (44) and the second C-clip is positioned externally of the first cavity of the gear casing and securing the rotating shaft to the gear casing.

8. A surgical stapling device (10) comprising:
a powered handle assembly (12) according to claim 1;
an adapter assembly (14) having a proximal portion (24) and a distal portion (26), the proximal portion coupled to the handle assembly, the adapter assembly including a firing rod (50) that is coupled to the rack and movable between retracted and advanced positions in response to movement of the rack between its retracted and advanced positions; and
a tool assembly (14) supported on the distal portion of the adapter assembly.

9. The stapling device of claim 8, wherein the handle assembly further includes a crank lever (80) coupled to the rotating shaft, the crank lever movable to move the spur gear from first position to the second position.

10. The stapling device of claim 9, wherein the handle assembly further includes a biasing member (120) engaged with the spur gear, the biasing member urging the spur gear towards the first position, optionally wherein the handle assembly further includes a first portion (104) and a second portion (108), the first portion rotatably fixed to the spur gear, and the second portion receiving the crank lever and preferably wherein the handle assembly further includes first and second C-clips (110, 112), the second portion of the rotating shaft defining spaced annular grooves (106, 108) that receive the first and second C-clips, respectively, the spur gear being received about the second portion of the rotating shaft atop the first C-clip within the first cavity of the gear casing and the second C-clip being positioned externally of the first cavity of the gear casing and securing the rotating shaft to the gear casing, yet more optionally.

11. The stapling device of claim 10, wherein the housing of the handle assembly defines an opening (40) and includes a removable cover (42) positioned over the opening, the crank lever being accessible through the opening.

12. The stapling device of claim 11, wherein the crank lever of the handle assembly is movable along the second portion of the rotating shaft from a first position in which the rotating shaft can rotate independently of the crank lever to a second position in which the crank lever is rotatably fixed to the rotating shaft.

13. The stapling device of claim 12, wherein the crank lever of the handle assembly includes a hub (84) that defines a through bore having a rectangular portion and the second portion of the rotating shaft includes a rectangular portion that is received within the rectangular portion of the through bore when the crank lever is in its second position.

14. The stapling device of claim 8, wherein the tool assembly includes an anvil and a cartridge assembly (150, 152), the anvil and the cartridge assembly cartridge assembly being movable between open and clamped positions.

15. The stapling device of claim 14, further including a drive assembly (158) coupled to the firing rod, the drive assembly including a working end having an I-beam configuration, the working end of the drive assembly movable in relation to the anvil and the cartridge assembly in response to movement of the firing rod between its retracted and advanced positions.

## Patentansprüche

1. Motorisierte Griffanordnung (12) für eine chirurgische Vorrichtung (10), die Folgendes umfasst:
ein Gehäuse (12), das einen Hohlraum (38) definiert;
einen Getriebekasten (44), der innerhalb des Hohlraums des Gehäuses getragen wird, wobei der Getriebekasten einen Längskanal (64), einen ersten Hohlraum (60) und einen zweiten Hohlraum (62) definiert, die miteinander kommunizieren;
eine Motoranordnung (46), die eine Abtriebswelle (70) und ein Antriebszahnrad (56) einschließt, das an der Abtriebswelle gesichert ist, wobei die Motoranordnung an dem Getriebekasten gesichert ist und das Antriebszahnrad innerhalb des zweiten Hohlraums des Getriebekastens positioniert ist;
eine Zahnstange (48), die in dem Längskanal des Getriebekastens aufgenommen ist und zwischen einer zurückgezogenen und einer vorgeschobenen Position bewegbar ist;
eine rotierende Welle (78), die sich durch den ersten Hohlraum des Getriebekastens erstreckt; und
ein Stirnrad (54), das mit der rotierenden Welle gekoppelt ist und innerhalb des ersten Hohlraums des Getriebekastens aufgenommen ist, wobei das Stirnrad innerhalb des ersten Hohlraums von einer ersten Position, in der das Stirnrad mit dem Antriebsrad und der Zahnstange in Eingriff ist, in eine zweite Position, in der das Stirnrad aus dem Eingriff mit dem Antriebsrad gelöst und mit der Zahnstange in Eingriff ist, bewegbar ist.

2. Motorisierter Griff nach Anspruch 1, der ferner einen Kurbelhebel (80) einschließt, der mit der rotierenden Welle gekoppelt ist, wobei der Kurbelhebel bewegbar ist, um das Stirnrad von der ersten Position in die zweite Position zu bewegen, gegebenenfalls ferner einschließlich eines Vorspannelements (120), das mit dem Stirnrad in Eingriff ist, wobei das Vorspannelement das Stirnrad in Richtung der ersten Position drängt.

3. Motorisierter Griff nach Anspruch 2, wobei die rotierende Welle einen ersten Abschnitt (104) und einen zweiten Abschnitt (108) einschließt, wobei der erste Abschnitt rotierbar an dem Stirnrad fixiert ist und der zweite Abschnitt den Kurbelhebel aufnimmt.

4. Motorisierter Griff nach Anspruch 3, wobei das Gehäuse eine Öffnung (40) definiert und eine entfernbare Abdeckung (42) einschließt, die über der Öffnung positioniert ist, wobei der Kurbelhebel durch die Öffnung zugänglich ist.

5. Motorisierter Griff nach Anspruch 4, wobei der Kurbelhebel entlang des zweiten Abschnitts der rotierenden Welle von einer ersten Position, in der die rotierende Welle unabhängig von dem Kurbelhebel rotieren kann, in eine zweite Position, in der der Kurbelhebel rotierbar an der rotierende Welle fixiert ist, bewegbar ist.

6. Motorisierter Griff nach Anspruch 5, wobei der Kurbelhebel eine Nabe (84) einschließt, die eine Durchgangsbohrung mit einem rechteckigen Abschnitt (86b) definiert, und der zweite Abschnitt der rotierende Welle einen rechteckigen Abschnitt (104) einschließt, der innerhalb des rechteckigen Abschnitts der Durchgangsbohrung aufgenommen wird, wenn sich der Kurbelhebel in seiner zweiten Position befindet.

7. Motorisierter Griff nach Anspruch 3, der ferner einen ersten und einen zweiten C-Clip (110, 112) einschließt, wobei der zweite Abschnitt der rotierenden Welle beabstandete ringförmige Nuten definiert, die den ersten bzw. zweiten C-Clip aufnehmen, wobei das Stirnrad um den zweiten Abschnitt der rotierenden Welle auf dem ersten C-Clip innerhalb des ersten Hohlraums des Getriebekastens (44) aufgenommen wird und der zweite C-Clip außerhalb des ersten Hohlraums des Getriebekastens positioniert ist und die rotierende Welle an dem Getriebekasten sichert.

8. Chirurgische Klammervorrichtung (10), die Folgendes umfasst:
eine motorisierte Griffanordnung (12) nach Anspruch 1;
eine Adapteranordnung (14) mit einem proximalen Abschnitt (24) und einem distalen Abschnitt (26), wobei der proximale Abschnitt mit der Griffanordnung gekoppelt ist, wobei die Adapteranordnung eine Auslösestange (50) einschließt, die mit der Zahnstange gekoppelt ist und in Reaktion auf eine Bewegung der Zahnstange zwischen ihrer zurückgezogenen und ihrer vorgeschobenen Position bewegbar ist; und
eine Werkzeuganordnung (14), die auf dem distalen Abschnitt der Adapteranordnung getragen wird.

9. Klammervorrichtung nach Anspruch 8, wobei die Griffanordnung ferner einen Kurbelhebel (80) einschließt, der mit der rotierenden Welle gekoppelt ist, wobei der Kurbelhebel bewegbar ist, um das Stirnrad von der ersten Position in die zweite Position zu bewegen.

10. Klammervorrichtung nach Anspruch 9, wobei die Griffanordnung ferner ein Vorspannelement (120) einschließt, das mit dem Stirnrad in Eingriff ist, wobei das Vorspannelement das Stirnrad in Richtung der ersten Position drängt, wobei gegebenenfalls die Griffanordnung ferner einen ersten Abschnitt (104) und einen zweiten Abschnitt (108) einschließt, wobei der erste Abschnitt rotierbar an dem Stirnrad fixiert ist und der zweite Abschnitt den Kurbelhebel aufnimmt, und wobei die Griffanordnung vorzugsweise ferner einen ersten und einen zweiten C-Clip (110, 112) einschließt, wobei der zweite Abschnitt der rotierende Welle beabstandete ringförmige Nuten (106, 108) definiert, die den ersten bzw. zweiten C-Clip aufnehmen, wobei das Stirnrad um den zweiten Abschnitt der rotierenden Welle auf dem ersten C-Clip innerhalb des ersten Hohlraums des Getriebekastens aufgenommen wird und der zweite C-Clip außerhalb des ersten Hohlraums des Getriebekastens positioniert ist und noch optionaler die rotierende Welle an dem Getriebekasten sichert.

11. Klammervorrichtung nach Anspruch 10, wobei das Gehäuse der Griffanordnung eine Öffnung (40) definiert und eine entfernbare Abdeckung (42) einschließt, die über der Öffnung positioniert ist, wobei der Kurbelhebel durch die Öffnung zugänglich ist.

12. Klammervorrichtung nach Anspruch 11, wobei der Kurbelhebel der Griffanordnung entlang des zweiten Abschnitts der rotierenden Welle von einer ersten Position, in der die rotierende Welle unabhängig von dem Kurbelhebel rotieren kann, in eine zweite Position bewegbar ist, in der der Kurbelhebel rotierbar an der rotierenden Welle fixiert ist.

13. Klammervorrichtung nach Anspruch 12, wobei der Kurbelhebel der Griffanordnung eine Nabe (84) einschließt, die eine Durchgangsbohrung mit einem rechteckigen Abschnitt definiert, und der zweite Abschnitt der rotierende Welle einen rechteckigen Abschnitt einschließt, der innerhalb des rechteckigen Abschnitts der Durchgangsbohrung aufgenommen wird, wenn sich der Kurbelhebel in seiner zweiten Position befindet.

14. Klammervorrichtung nach Anspruch 8, wobei die Werkzeuganordnung einen Amboss und eine Magazinanordnung (150, 152) einschließt, wobei der Amboss und die Magazinanordnung zwischen offenen und geklemmten Positionen bewegbar sind.

15. Klammervorrichtung nach Anspruch 14, die ferner eine Antriebsanordnung (158) einschließt, die mit der Auslösestange gekoppelt ist, wobei die Antriebsanordnung ein Arbeitsende mit einer I-Träger-Konfiguration umfasst, wobei das Arbeitsende der Antriebsanordnung in Bezug auf den Amboss und die Magazinanordnung in Reaktion auf Bewegung der Auslösestange zwischen ihrer zurückgezogenen und ihrer vorgeschobenen Position bewegbar ist.

## Revendications

1. Ensemble de poignée motorisée (12) pour un dispositif chirurgical (10) comprenant :
un logement (12) définissant une cavité (38) ;
un carter d'engrenage (44) supporté à l'intérieur de la cavité du logement, le carter d'engrenage définissant un canal longitudinal (64), une première cavité (60), et une seconde cavité (62) communiquant entre elles ;
un ensemble moteur (46) comprenant un arbre de sortie (70) et un engrenage d'entraînement (56) fixé à l'arbre de sortie, l'ensemble moteur fixé au carter d'engrenage et l'engrenage d'entraînement étant positionné à l'intérieur de la seconde cavité du carter d'engrenage ;
une crémaillère (48) reçue à l'intérieur du canal longitudinal du carter d'engrenage et mobile entre des positions rétractée et avancée ;
un arbre rotatif (78) s'étendant à travers la première cavité du carter d'engrenage ; et
un engrenage droit (54) couplé à l'arbre rotatif et reçu dans la première cavité du carter d'engrenage, l'engrenage droit pouvant se déplacer dans la première cavité d'une première position dans laquelle l'engrenage droit est en prise avec l'engrenage d'entraînement et la crémaillère à une seconde position dans laquelle l'engrenage droit est libéré de l'engrenage d'entraînement et en prise avec la crémaillère.

2. Poignée motorisée selon la revendication 1, comprenant en outre un levier à manivelle (80) couplé à l'arbre rotatif, le levier à manivelle étant mobile pour déplacer l'engrenage droit de la première position à la seconde position, comprenant en outre facultativement un élément de sollicitation (120) en prise avec l'engrenage droit, l'élément de sollicitation poussant l'engrenage droit vers la première position.

3. Poignée motorisée selon la revendication 2, l'arbre rotatif comprenant une première partie (104) et une seconde partie (108), la première partie étant fixée de manière rotative à l'engrenage droit, et la seconde partie recevant le levier à manivelle.

4. Poignée motorisée selon la revendication 3, le logement définissant une ouverture (40) et comprenant un couvercle amovible (42) positionné au-dessus de l'ouverture, le levier à manivelle étant accessible à travers l'ouverture.

5. Poignée motorisée selon la revendication 4, le levier à manivelle étant mobile le long de la seconde partie de l'arbre rotatif d'une première position dans laquelle l'arbre rotatif peut tourner indépendamment du levier à manivelle à une seconde position dans laquelle le levier à manivelle est fixé de manière rotative à l'arbre rotatif.

6. Poignée motorisée selon la revendication 5, le levier à manivelle comprenant un moyeu (84) qui définit un alésage traversant ayant une partie rectangulaire (86b) et la seconde partie de l'arbre rotatif comprenant une partie rectangulaire (104) qui est reçue dans la partie rectangulaire de l'alésage traversant lorsque le levier à manivelle est dans sa seconde position.

7. Poignée motorisée selon la revendication 3, comprenant en outre des premier et second attaches en C (110, 112), la seconde partie de l'arbre rotatif définissant des rainures annulaires espacées qui reçoivent les premier et second attaches en C, respectivement, l'engrenage droit étant reçu autour de la seconde partie de l'arbre rotatif au-dessus de la première attache en C à l'intérieur de la première cavité du carter d'engrenage (44) et la seconde attache en C étant positionnée à l'extérieur de la première cavité du carter d'engrenage et fixant l'arbre rotatif au carter d'engrenage.

8. Dispositif d'agrafage chirurgical (10) comprenant :
un ensemble de poignée motorisée (12) selon la revendication 1 ;
un ensemble adaptateur (14) comportant une partie proximale (24) et une partie distale (26), la partie proximale étant couplée à l'ensemble poignée, l'ensemble adaptateur comprenant une tige de déclenchement (50) couplée à la crémaillère et mobile entre des positions rétractée et avancée en réponse au mouvement de la crémaillère entre ses positions rétractée et avancée ; et
un ensemble outil (14) supporté sur la partie distale de l'ensemble adaptateur.

9. Dispositif d'agrafage selon la revendication 8, l'ensemble de poignée comprenant en outre un levier à manivelle (80) couplé à l'arbre rotatif, le levier à manivelle pouvant être déplacé pour déplacer l'engrenage droit de la première position à la seconde position.

10. Dispositif d'agrafage selon la revendication 9, l'ensemble de poignée comprenant en outre un élément de sollicitation (120) engagé avec l'engrenage droit, l'élément de sollicitation poussant l'engrenage droit vers la première position, facultativement l'ensemble de poignée comprenant en outre une première partie (104) et une seconde partie (108), la première partie étant fixée de manière rotative à l'engrenage droit, et la seconde partie recevant le levier à manivelle et de préférence l'ensemble de poignée comprenant en outre des première et seconde attaches en C (110, 112), la seconde partie de l'arbre rotatif définissant des rainures annulaires espacées (106, 108) qui reçoivent respectivement les première et seconde attaches en C, l'engrenage droit étant reçu autour de la seconde partie de l'arbre rotatif au-dessus de la première attache en C à l'intérieur de la première cavité du carter d'engrenage et la seconde attache en C étant positionnée à l'extérieur de la première cavité du carter d'engrenage et fixant l'arbre rotatif au carter d'engrenage, encore plus facultativement.

11. Dispositif d'agrafage selon la revendication 10, le logement de l'ensemble de poignée définissant une ouverture (40) et comprenant un couvercle amovible (42) positionné au-dessus de l'ouverture, le levier à manivelle étant accessible à travers l'ouverture.

12. Dispositif d'agrafage selon la revendication 11, le levier à manivelle de l'ensemble de poignée étant mobile le long de la seconde partie de l'arbre rotatif d'une première position dans laquelle l'arbre rotatif peut tourner indépendamment du levier à manivelle à une seconde position dans laquelle le levier à manivelle est fixé de manière rotative à l'arbre rotatif.

13. Dispositif d'agrafage selon la revendication 12, le levier à manivelle de l'ensemble de poignée comprenant un moyeu (84) qui définit un alésage traversant ayant une partie rectangulaire et la seconde partie de l'arbre rotatif comprenant une partie rectangulaire qui est reçue dans la partie rectangulaire de l'alésage traversant lorsque le levier à manivelle est dans sa seconde position.

14. Dispositif d'agrafage selon la revendication 8, l'ensemble d'outils comprenant une enclume et un ensemble de cartouche (150, 152), l'enclume et l'ensemble de cartouche étant mobile entre des positions ouverte et serrée.

15. Dispositif d'agrafage selon la revendication 14, comprenant en outre un ensemble d'entraînement (158) couplé à la tige de déclenchement, l'ensemble d'entraînement comprenant une extrémité de travail ayant une configuration de poutre en I, l'extrémité de travail de l'ensemble d'entraînement pouvant être déplacée par rapport à l'enclume et à l'ensemble de cartouche en réponse au déplacement de la tige de déclenchement entre ses positions rétractée et avancée.
